(19) Europäisches Patentamt
European Patent Office
Office européen des brevets

(11) Numéro de publication: **0 286 478 B1**

(12) **FASCICULE DE BREVET EUROPEEN**

(45) Date de publication de fascicule du brevet: **10.03.93**

(51) Int. Cl.$^5$: **C07D 251/54**, C07D 251/70, C07D 251/48, C07D 235/30, C07D 249/08, C07D 249/14, C07D 295/02, C08K 5/34

(21) Numéro de dépôt: **88400619.8**

(22) Date de dépôt: **15.03.88**

Le dossier contient des informations techniques présentées postérieurement au dépôt de la demande et ne figurant pas dans le présent fascicule.

(54) **Sels d'acide phosphoreux, compositions les contenant et leur application comme agent d'ignifugation.**

(30) Priorité: **31.03.87 FR 8704488**

(43) Date de publication de la demande:
**12.10.88 Bulletin 88/41**

(45) Mention de la délivrance du brevet:
**10.03.93 Bulletin 93/10**

(84) Etats contractants désignés:
**AT BE CH DE ES FR GB GR IT LI LU NL SE**

(56) Documents cités:
**EP-A- 0 149 480**
**FR-A- 2 246 586**
**US-A- 3 810 850**
**US-A- 4 119 724**
**US-A- 4 574 154**

**CHEMICAL ABSTRACTS, vol. 89, no. 14, 2 octobre 1978, page 87, résumé no. 111478u, Columbus, Ohio; & JP-A-78 49 054 (ASAHI CHEMICAL INDUSTRY CO., LTD) 04-05-1978**

(73) Titulaire: **ELF ATOCHEM S.A.**
**4 & 8, Cours Michelet La Défense 10**
**F-92800 Puteaux(FR)**

(72) Inventeur: **Poisson, Pierre**
**Le Mascrier**
**F-27300 Bernay(FR)**
Inventeur: **Rivas, Nadine**
**Rue des Jonquilles Cedex 28-7**
**F-27670 Saint-Ouen du Tilleul(FR)**
Inventeur: **Deloy, Pierre**
**12 Allée Youri Gagarine**
**F-92300 Levallois-Perret(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 286 478 B1

## Description

La présente invention concerne de nouveaux sels de l'acide phosphoreux. Ces produits sont utiles comme ignifugeants des matières plastiques.

On connaît de nombreux dérivés du phosphore, notamment des phosphites et des phosphonates qui trouvent une application comme retardateurs de flamme dans de nombreuses matières inflammables. Le brevet US 4 499 222 décrit des alkyls phosphonates halogénés utilisés principalement dans les polyuréthanes.

L'amélioration du comportement au feu des polyuréthanes peut également être réalisé par l'emploi de phosphates de piperazine -brevet US 3 810 850- ou bien de sels d'amine d'acide phosphoniques -brevet EP 149 480.

La demande de brevet Japonais 51-123 884 déposée le 18 octobre 1976 décrit une composition de résine polyamide ayant un comportement au feu amélioré contenant une charge inorganique minérale (silicate de calcium, talc, carbonate de calcium, ...) et un sel de mélamine d'acide phosphorique dont la proportion d'acide phosphorique peut varier de 28 % à 55 %.

On a maintenant trouvé de nouveaux dérivés de l'acide phosphoreux qui sont utiles comme retardateurs de flamme dans les matières plastiques.

Plus précisément, la présente invention concerne de nouveau sels d'acide phosphoreux de formule :

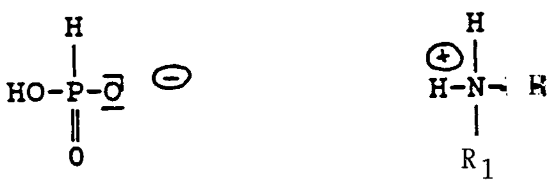

(I)

dans laquelle

$R_1$ un radical hétérocyclique, éventuellement substitué par des halogènes, des groupements amino, des radicaux phényles, cycliques ou alkyles. L'invention concerne également des produits de formule:

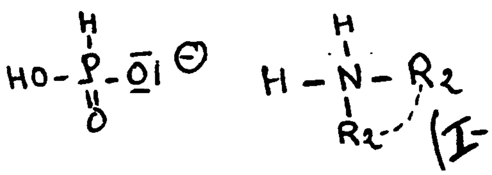

(I-a)

dans laquelle les substituants $R_2$ sont des restes aliphatiques identiques ayant jusqu'a 4 atomes de carbone pouvant être substitues par des halogènes ou des groupements amino, ces restes $R_2$ étant pontés par un groupement X qui peut être un reste > NH ou un reste méthylénique -CH2-.

La présente invention concerne aussi des produits de formule (II)

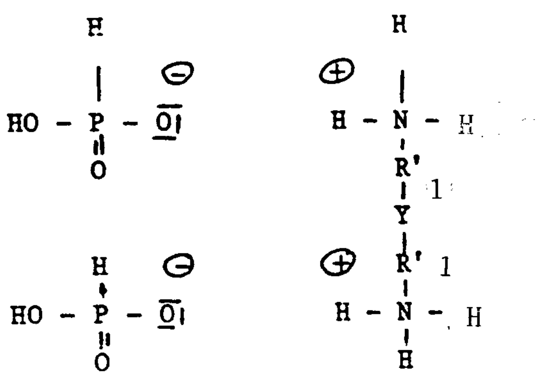

II

dans laquelle $R_1'$ est un radical hétérocyclique éventuellement substitué par des halogènes, des groupements amino, des radicaux phényles, cycliques ou alkyles, Y est un groupement :

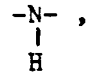

$-NH(CH_2)_n-NH-$ avec n compris entre 2 et 6,

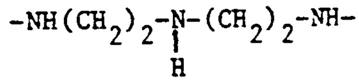

ou

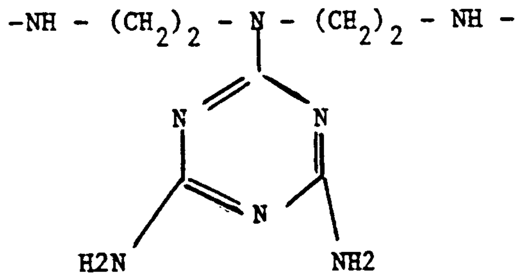

ou une simple liaison.

On utilise avantageusement des produits de formule (I) et (I-a).

Les produits de formule (I) dans laquelle $R_1$ est un hétérocycle contenant des atomes d'azote et pouvant être substitué par des groupements amino, des halogènes, des radicaux aliphatiques ayant jusqu'à 10 atomes de carbone, des radicaux phényles. On préfère les produits dans lesquels $R_1$ est un des hétérocycles suivants :

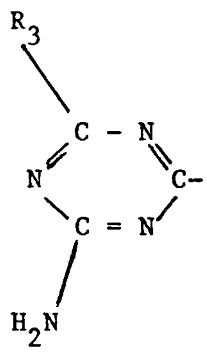

(Structure s-triazinique)

dans laquelle $R_3$ peut être un radical amino, un radical hydrocarboné aliphatique ayant un nombre d'atomes de carbone compris entre 1 et 10, un radical phényle substitué ou non.

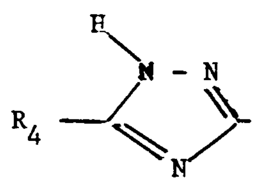

(Structure-1,2,4 triazolyle)

et ses isomères dans laquelle $R_4$ peut être un hydrogène, un radical amino.

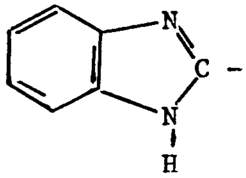

(Structure benzimidazolyle)

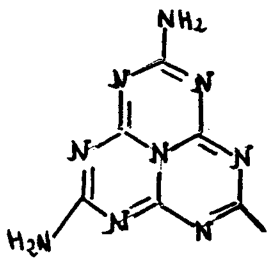

(Structure heptazinique)

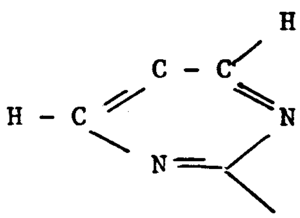

(Structure 1,3 diazinique ou pyrimidinique)

On utilise plus particulièrement dans cette première famille :

- le phosphite de mélamine :

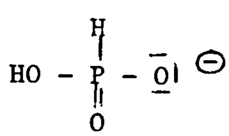

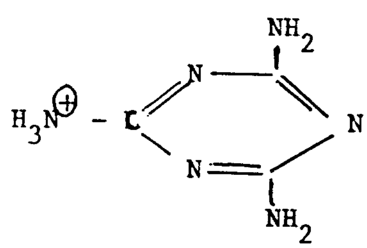

- le phosphite de melem :

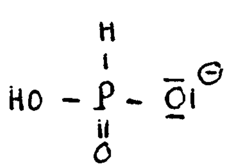

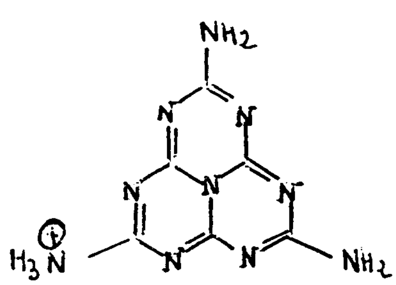

- le phosphite de benzoguanamine :

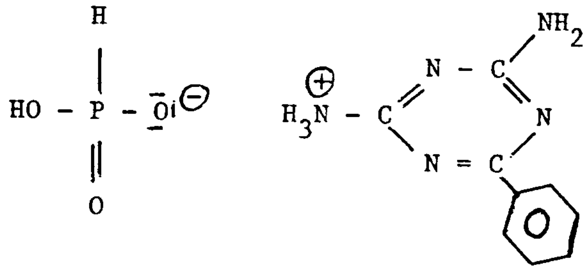

- le phosphite d'acétoguanamine :

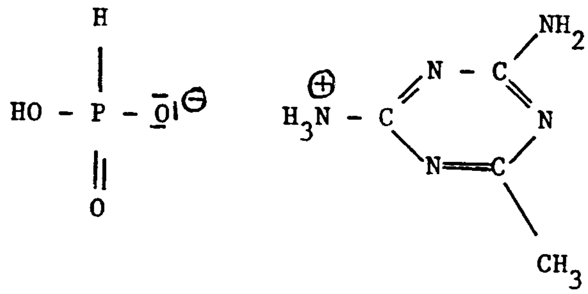

- le phosphite d'amino-3 triazole-1,2,4 :

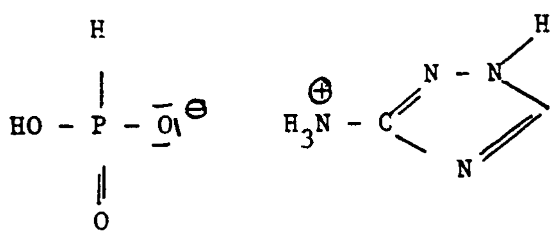

- le phosphite d'amino-4 triazole-1,2,4 :

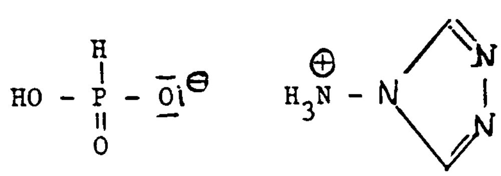

- le phosphite de guanazole :

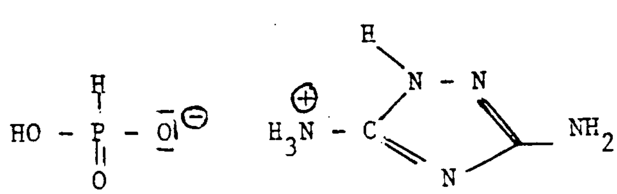

- le phosphite de benzimidazole :

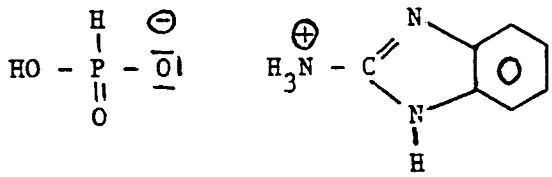

Les produits de formule (I-a) dans laquelle $R_2$ est un reste aliphatique ayant jusqu'à 4 atomes de carbone pouvant être substitué par des halogènes ou des groupements amino, les deux restes $R_2$ étant pontés par un reste > NH ou un reste méthylénique.

Dans les produits de formule (I-a), on préfère les restes $R_2$ tels qu'on ait une structure piperazinique ou pipéridinique. On utilise plus particulièrement :

- le phosphite de pipérazine :

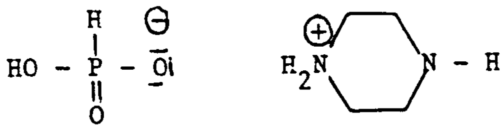

- le phosphite de piperidine :

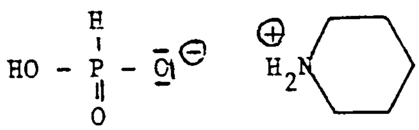

Parmi les produits de formule (II), on utilise avantageusement ceux dans lesquels R'$_1$ un hétérocycle contenant des atomes d'azote et pouvant être substitué par des groupements amino, des halogènes, des radicaux aliphatiques ayant jusqu'à 10 atomes de carbone. On préfère les produits dans lesquels R'$_1$ une structure triazinique telle que :

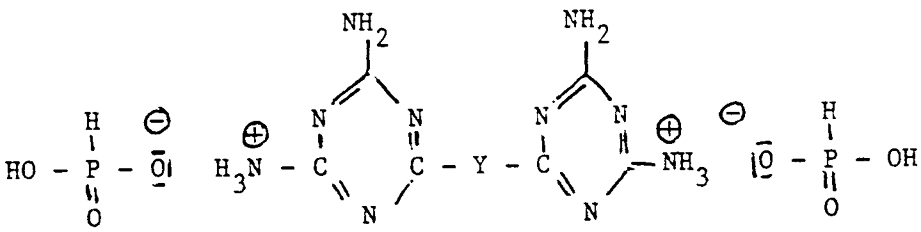

On utilise plus particulièrement :

- le diphosphite de mélam :

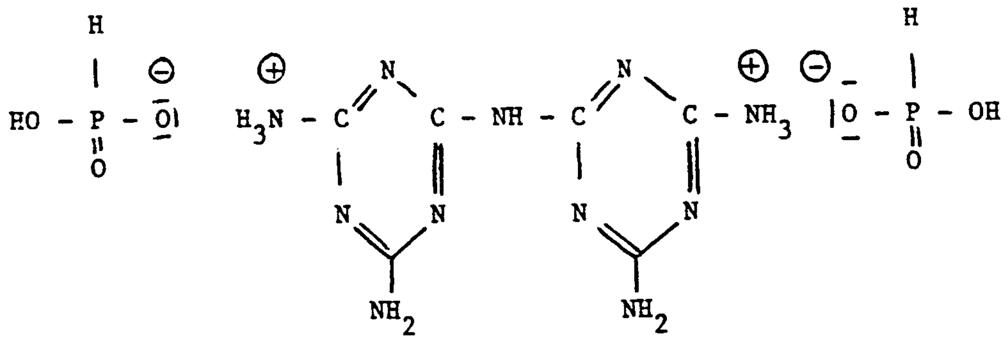

- le diphosphite d'éthylène dimélamine :

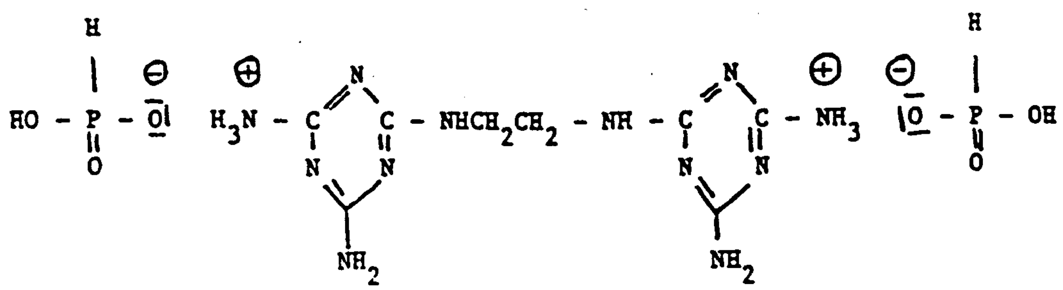

- le diphosphite du diethylènetrimelamine :

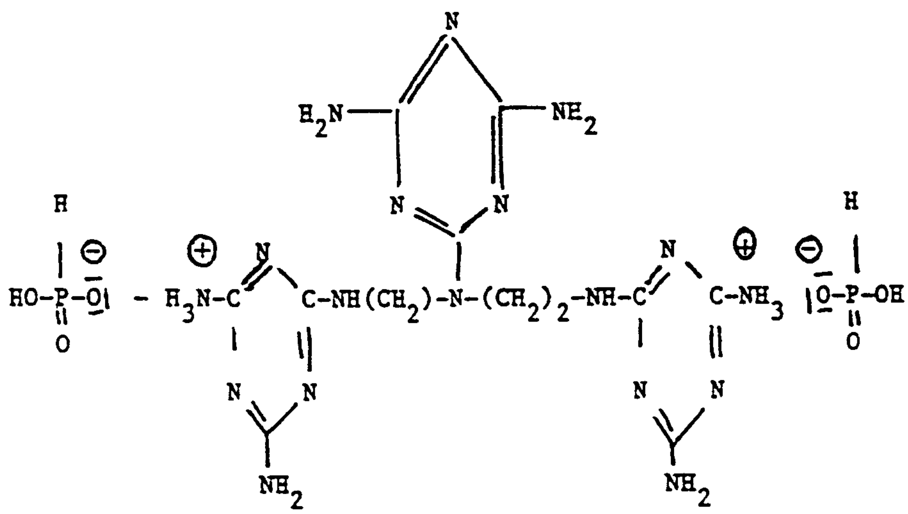

On peut également utiliser avantageusement des prouduits de formule:

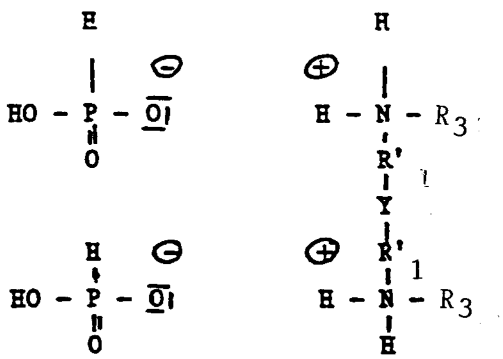

(II-b)

dans laquelle les deux radicaux $R_3$ sont des restes aliphatiques ayant la 2 atomes de carbone et sont liés par une simple liaison et y une simple liaison, le produit étant de préférence le diphosphite de pipérazine :

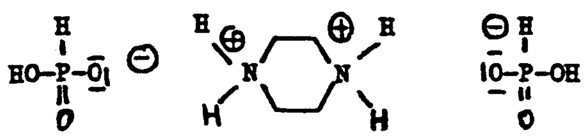

Les composés de la présente invention peuvent être obtenus par divers moyens.

On peut faire réagir ensemble un composé de structure

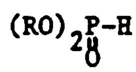

dans lequel R peut être $CH_3$ ou $CH_3CH_2$-, avec un composé de formule générale :

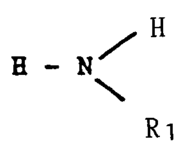

$R_1$ ayant la même signification que précédemment et de l'eau. Ce procédé est long et peu économique. On a trouvé qu'il était préférable de faire réagir en milieu aqueux l'acide phosphoreux (sous forme de solution aqueuse) avec un composé de structure

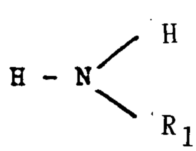

selon la réaction :

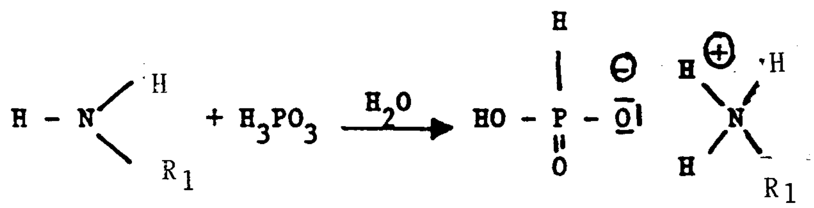

Avantageusement le rapport molaire

$$\frac{\text{acide phosphoreux}}{\text{amine}}$$

est compris entre 0,9 et 1,1 mais de préférence égal à 1.

Parmi les amines qu'on peut utiliser selon l'invention, on peut citer par exemple la mélamine, la benzoguanamine, l'acétoguanamine, la diamino-2,4 nonyl-6 triazine-1,3,5, l'amino-3 triazole-1,2,4, le guanazole, le mélam, le mélem, la piperazine, l'amino-2 benzimidazole, le bis(triazinyl-1,3,5triamino-2,4,6)-N,N'éthane-1,2.

Un mode de préparation des phospbites d'amines préfèré selon l'invention consiste à traiter une suspension aqueuse d'amine par une solution aqueuse d'acide phosphoreux de concentration comprise entre 60 et 80 %, de préférence voisine de 70 % en poids. La réaction peut être réalisée entre 20 et 100 ° C, de préférence vers 30 à 50 ° C, pendant une durée qui peut aller de une à plusieurs heures sous bonne agitation.

Les phosphites d'amines ainsi obtenus sont peu ou pas solubles dans le milieu réactionnel. On peut réutiliser avantageusement le filtrat pour une opération ultérieure, ce qui permet d'améliorer le rendement.

Pour isoler le composé, on procède par les moyens connus : essorage, lavage du gâteau obtenu, séchage vers 100 ° C sous pression réduite.

Avantageusement on broie le produit pour obtenir des poudres de granulométrie adéquate pour permettre une bonne dispersion dans la matrice polymérique à ignifuger. Avantageusement la granulométrie est comprise entre 1 et 50 microns et de préférence comprise entre 5 et 25 microns.

Les produits sont caractérisés par analyse élémentaire, infrarouge, RMN du proton, du carbone 13 et du phosphore 31.

La pureté peut être rapidement déterminée par un simple dosage acidimétrique.

Les phosphites d'amines sont des produits stables à des températures inférieures à 250 ° C. Les phosphites d'amines ainsi obtenus peuvent être utilisés comme ignifugeants de matières organiques et en particulier de matières plastiques.

La présente invention concerne aussi l'application des produits de la présente invention comme agents d'ignifugation. La présente invention concerne aussi l'application des phosphites de morpholine, cyclohexylamine et d'aniline comme agent d'ignifugation.

Avantageusement ces produits sont utilisés pour ignifuger les polyamides et les polyoléfines.

Par "polyamides" nous entendons les polymères résultant de la polycondensation d'un ou plusieurs aminoacides tels que les acides aminocaproïques, amino-7 heptanoïque, amino-11 undécanoïque .... d'un ou plusieurs lactames, tels que le caprolactame, le lauryllactame ... d'un ou plusieurs sels ou mélanges de diamines telles l'hexamethylène diamine, la dodécaméthylènediamine avec des diacides tels que les acides téréphtalique, adipique, azélaïque, ....... ou des mélanges de tous ces monomères, ce qui conduit à des copolyamides.

On utilise les phosphites d'amine de la présente invention avantageusement à raison de 1 à 20 % en poids par rapport au polyamide ignifugé et, de préférence, en proportion de 3 à 12 %.

Par "polyoléfines", nous entendons tous les polymères de monooléfines qui répondent à la formule $CH_2$ = CH - A dans laquelle A représente un hydrogène, un radical hydrocarboné substitué ou non ayant un nombre de carbone compris entre 1 et 10, un radical phényle, un radical acétoxy.

De tels polymères qu'on peut ignifuger selon l'invention sont les polyéthylènes, les copolymères éthylène-propylène, le polypropylène, le poly(acétate de vinyle).

On utilise les phosphites d'amines de la présente invention avantageusement à raison de 10 à 60 % en poids par rapport aux polyoléfines ignifugées, et de préférence en proportion de 25 à 35 %.

Selon un mode préféré de mise en ouvre des produits de l'invention, on les utilise dans des systèmes intumescents.

Un système intumescent se caractérise par le fait qu'au moment de la combustion les composés dudit système interréagissent pour former une mousse carbonacée plus ou moins alvéolaire incombustible qui ralentit la libération des gaz inflammables libérés de la masse chauffée. De tels systèmes sont constitués d'une façon générale de trois constituants principaux :

- un spumogène qui produit des gaz ou des vapeurs ininflammables qui aident à la formation de la mousse. Ce sont généralement des composés azotés tels que urée, guanidine, mélamine...
- un agent de carbonisation qui contribue à la formation de matières carbonacées ("chars"). Ce sont généralement des composés polyhydroxyles tels que sucres, mono, di ou tripentaerythrite, trimethylolpropane...
- un catalyseur qui est généralement un composé acide ou, plus précisément, un composé générateur d'acide au moment de la combustion. C'est-à-dire que cet acide est le plus souvent sous forme combinée. Les catalyseurs les plus couramment employés sont les polyphosphates d'ammonium, les phosphates de mélamine, les borates de mélamine et les sulfates de mélamine. Comme on peut le remarquer nous avons dans la plupart des combinaisons, à la fois le spumogène et l'acide sous forme combinée.

Les phosphites d'amines de la présente invention peuvent également jouer à la fois le rôle de spumogène de par la présence du composé azoté et le rôle de catalyseur, de par la présence d'acide phosphoreux combiné.

Ils présentent l'avantage, par rapport aux combinaisons précitées, d'être des combinaisons bien définies, peu ou pas hygroscopiques, faciles à obtenir à partir de réactifs peu côuteux, et d'avoir un caractère plus réducteur que les dérivés des acides phosphoriques, d'ou une très grande efficacité lors de la combustion.

Les phosphites d'amines de la présente invention associés à des composés polyhydroxylés conduisent à des systèmes intumescents très efficaces qui permettent d'améliorer le comportement au feu des matières plastiques, en particulier des polyoléfines et des polyamides, lesdits polyamides et polyoléfines ayant été définis précédemment.

Les composés polyhydroxyles qui peuvent convenir sont l'erythrytol, le sorbitol, le mannitol, le dianhydrosorbitol, l'anhydroerythritol, la mono-di ou tripentaerythrite. De préférence on utilise la monopentaerythrite, qu'on appelle dans la suite du texte "pentaerythritol".

Comme phosphites d'amines, on peut utiliser tous les phosphites d'amines de la présente invention mais de préférence on utilise le phosphite de mélamine.

S'agissant des polyamides, on ajoute en plus des phosphites d'amines de l'invention, un pourcentage variable d'un ou plusieurs composés polyhydroxylés compris entre 0,2 et 10 % en poids par rapport aux polyamides ignifugées, et de préférence entre 1 et 3 %.

S'agissant des polyoléfines, les proportions des constituants définis par le rapport molaire

$$R = \frac{\text{phosphite d'amine}}{\text{composé polyhydroxyle}}$$

peut être compris entre 1 et 7, de préférence entre 2 et 4.

Comme pour les polyamides, le composé polyhydroxylé peut être un ou plusieurs produits.

La quantité des constituants - phosphite d'amine plus composé polyhydroxylé - défini comme le taux de charges, est fonction du degré d'ignifugation désiré.

Il peut être compris entre 20 et 60 % en poids par rapport à la résine ignifugée. D'une façon générale on obtient un comportement au feu amélioré et une bonne conservation des propriétés mécaniques, avec un taux de charges compris entre 25 et 35 %.

L'incorporation des phosphites d'amine et du composé polyhydroxylé s'effectue par malaxage de ces deux composés, finement divisés dans le polymère fondu - tout appareil de malaxage assurant une bonne dispersion peut donc convenir - conviennent particulièrement bien à cet effet les malaxeurs type BUSS.

Les conditions d'extrusion doivent être appropriées pour obtenir une bonne dispersion des additifs.

Le compound obtenu est granulé.

Les granulés obtenus sont moulés par injection ou par compression à des températures convenables en éprouvettes normalisées pour pratiquer l'essai de réaction au feu UL 94 selon la norme NF T51072 et pour mesurer l'indice d'oxygène selon la norme NF T51071.

Une manière simple d'opérer consiste à mélanger à sec les granulés de polymère, le phosphite d'amine et le composé polyhydroxylé dans un mélangeur type TURBULA ou plus simplement au tonneau et à alimenter avec ce mélange un malaxeur approprié.

On peut également préparer des mélanges maîtres polymère - phosphite d'amine. Avec ce compound sous forme de granulés on peut réaliser comme précédemment dans un mélangeur type TURBULA un mélange avec le composé polyhydroxylé puis alimenter un malaxeur. On peut également alimenter une extrudeuse en tête de vis malaxeuse avec un mélange polymère - phosphite d'amine puis au moyen d'un doseur type SODER introduire en milieu de vis le composé polyhydroxylé.

Les exemples suivants explicitent l'invention.

EXEMPLE 1

Phosphite de mélamine

Dans un réacteur de 250 ml muni d'une agitation d'une prise de température, d'un réfrigérant à reflux, on introduit :

12,6 g de mélamine (0,1 mole)
50 ml de diethyl phosphite
et 10 g d'eau,

puis on chauffe l'ensemble au bain d'eau bouillante sous bonne agitation.

Pendant la première heure de chauffe, on obtient une bouillie blanche qui devient de plus en limpide, voire transparente. Le chauffage de la solution ainsi obtenu est poursuivi et on observe la formation d'un précipité et un léger moussage. Il se produit un reflux important. Après 2 heures de chauffage on adapte un appareil à distiller, on récupère un produit de point d'ébullition égal à 78°C. La réaction est arrêtée lorsqu'il ne distille plus de produit.

On filtre à chaud, essore et lave le gâteau avec de l'éther. Le produit essoré est séché sous vide.

On obtient 18 g de phosphite de mélamine.

Rendement : 86,5 % exprimé par rapport à la mélamine mise en oeuvre.

Analyse élémentaire

| $C_3H_9N_6PO_3$ | | | | |
|---|---|---|---|---|
| | C | H | N | P |
| % calculé<br>% trouvé | 17,3<br>17,3 | 4,32<br>4,6 | 40,38<br>39,94 | 14,90<br>13,31 |

Spectre infra-rouge

$\nu$ P - H = 2400 $cm^{-1}$
$\nu$ P - OH = 2700 $cm^{-1}$
$\nu$ C = N (cycle) = 1620 $cm^{-1}$, 1520 $cm^{-1}$
$\nu$ $NH_3^{\oplus}$ = 3140 $cm^{-1}$
$\nu$ $NH_2$ = 3420 $cm^{-1}$
$\delta$ $NH_3^{\oplus}$ = 1405 $cm^{-1}$

Spectre RMN du Proton (solvant DMSO deutéré)

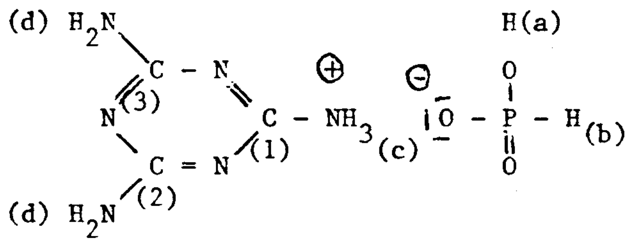

$\delta$ = 10,1 ppm (s) 1 Ha
$\delta$ = 6,75 ppm (d) 1 Hb JP - $H_{(b)}$ = 600 $H_z$
$\delta$ = 7,47 ppm (s) 3 $H_{(c)}$ + 4 $H_{(d)}$

Spectre RMN du $^{13}C$ (Solvant DMSO deutéré)

$\delta$ = 162,1 ppm $C_1$ + $C_2$ + $C_3$

Spectre RMN du $^{31}P$ (Solvant DMSO deutéré)

$\delta$ = 5,7 ppm

Stabilité thermique : $\Delta P$ = 2,98 % à 250 ° C.

EXEMPLE 2

Phosphite de mélamine

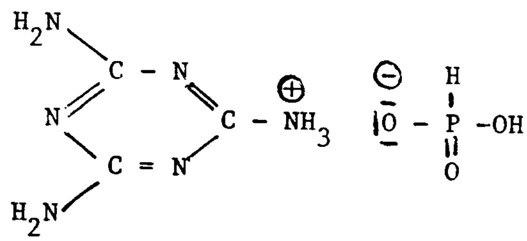

Dans un réacteur de 2 l muni d'une agitation, d'une prise de température, d'une ampoule d'addition et d'un réfrigérant ascendant, on introduit 1 l d'eau distillée et 126 g de mélamine (1 mole).

On agite vigoureusement de façon à disperser la mélamine puis on ajoute 117,15 g d'une solution aqueuse d'acide phosphoreux à 70 % (82 g $H_3PO_3$ : 1 mole) en 30 minutes. L'addition terminée on maintient le milieu réactionnel à température ambiante sous bonne agitation pendant 3 heures.

On essore, lave et sèche sous pression réduite à 100/120 ° C. On obtient 171 g de phosphite de mélamine.
Rendement : 82 %

Analyse élémentaire

| $C_3H_9N_6PO_3$ | | | | |
|---|---|---|---|---|
| | C | H | N | P |
| % calculé<br>% trouvé | 17,3<br>17,7 | 4,32<br>4,23 | 40,38<br>40,76 | 14,90<br>14,02 |

Spectre infra-rouge

$\nu$ P - H = 2356 $cm^{-1}$
$\nu$ P - OH = 2700 $cm^{-1}$
$\nu$ $NH_3^{\oplus}$ = 3125 $cm^{-1}$
$\nu$ P = O = 1078 $cm^{-1}$

Spectre RMN du Proton (Solvant DMSO deuteré)

$\nu$ = 6,62 ppm P - H JP - H = 612 $H_z$

Spectre RMN du $^{13}C$ (Solvant DMSO deuteré)

$\delta$ = 162,1 ppm

EXEMPLE 3

Préparation du phosphite de mélamine d'une façon analogue à l'exemple 2 sauf qu'on divise par deux la quantité d'eau : 500 ml pour 1 mole de mélamine.
Rendement : 82 %

Analyse élémentaire

| $C_3H_9N_6PO_3$ | | | | |
|---|---|---|---|---|
| | C | H | N | P |
| % calculé<br>% trouvé | 17,3<br>17,13 | 4,32<br>4,29 | 40,38<br>40,12 | 14,90<br>14,66 |

Spectre infra-rouge

$\nu$ P - H = 2400 $cm^{-1}$

Spectre RMN du $^{13}C$ (Solvant DMSO deuteré)

$\delta$ = 162,33 ppm

Spectre RMN du Proton (Solvant DMSO deuteré)

$\delta$ = 9,76 ppm P - O - H
$\delta$ = 6,75 ppm P - H JP - H = 600 $H_z$
$\delta$ = 7,49 ppm $NH_2$, $NH_3^{\oplus}$

EXEMPLE 4

Phosphite de benzoguanamine

Dans un réacteur de 2 l équipé comme dans l'exemple 2, on introduit 93,6 g de benzoguanamine (0,5 mole) dans 600 ml d'eau, on agite efficacement pour obtenir une bonne dispersion et introduit 62,2 g d'une solution aqueuse de $H_3PO_3$ à 72,5 % (0,55 mole $H_3PO_3$ 100 %).

L'addition terminée on porte 1 h 30 à 70 ° C. On refroidit, on filtre.

Le gâteau est empâté deux fois avec 300 ml d'eau, essoré puis séché à 80 ° C sous pression réduite jusqu'à poids constant.

On obtient 127 g de phosphite de benzoguanamine.

Rendement : 94,3 %

Analyse élémentaire

| $C_9H_{12}N_5PO_3$ | | | | |
|---|---|---|---|---|
| | C | H | N | P |
| % calculé<br>% trouvé | 40,14<br>40,35 | 4,46<br>4,52 | 26,02<br>26,15 | 11,52<br>11,3 |

Spectre infra-rouge

$\nu$ P - H = 2300 $cm^{-1}$
$\nu$ P - OH = 2700 - 2800 $cm^{-1}$
$\nu$ $CH_{ar}$ = 3060 $cm^{-1}$
$\nu$ $NH_3^{\oplus}$ = 3160 $cm^{-1}$
$\nu$ $NH_2$ = 3380 $cm^{-1}$

Spectre RMN du Proton (Solvant DMSO deuteré)

$\delta$ = 6,67 ppm P - H JP - H = 631 $H_z$

Spectre RMN du $^{13}C$ (Solvant DMSO deuteré)

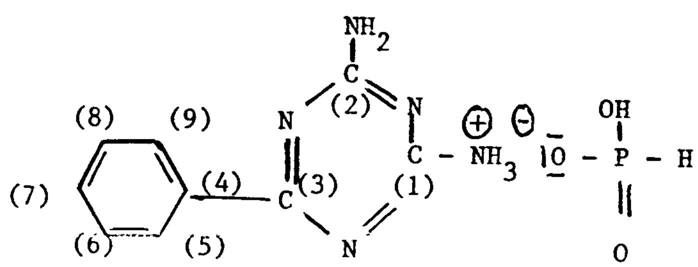

$\delta$ = 166,75 ppm $C_1$ - $C_2$
$\delta$ = 169,76 ppm $C_3$
$\delta$ = 136,46 ppm $C_4$
$\delta$ = 128,76 ppm $C_5$ - $C_9$
$\delta$ = 127,93 ppm $C_6$ - $C_8$
$\delta$ = 131,43 ppm $C_7$

EXEMPLE 5

Phosphite de guanazole

Dans un appareil identique à l'exemple 2 on introduit 500 ml d'eau et 599,3 g de guanazole (6,05 moles). On agite la suspension et introduit à température ambiante 720 g d'une solution aqueuse à 69 % de $H_3PO_3$ (6,05 moles).

Pendant l'addition qui dure 70 minutes le milieu passe par un stade homogène et la température s'élève de 20 à 35 °C.

En fin d'addition on observe un débit de cristallisation. On maintient le milieu à 30/35 °C pendant 1 h 30 sous bonne agitation. Puis on refroidit vers 10 °C et on essore les cristaux obtenus. On lave avec le minimum d'eau froide. On sèche sous vide à 140 °C.

On obtient 853 g de phosphite de guanazole qui se présente sous forme de cristaux blancs qui fondent à 163,2 °C.

Rendement : 77,82 %

Analyse élémentaire

| | C | H | N |
|---|---|---|---|
| % calculé | 13,25 | 4,41 | 38,67 |
| % trouvé | 12,6 | 4,38 | 38,22 |

Spectre infra-rouge

$\nu$ P - H = 2200 $cm^{-1}$
$\nu$ P - OH = 2680 $cm^{-1}$
$\nu$ $NH_2$, $NH_3^{\oplus}$ = 3080 $cm^{-1}$, 3160 $cm^{-1}$, 3360 $cm^{-1}$

Spectre RMN du Proton (Solvant DMSO deuteré)

$\delta$ = 6,65 ppm P - H JP - H = 619 $H_z$

Spectre RMN du $^{13}C$ (Solvant DMSO deuteré)

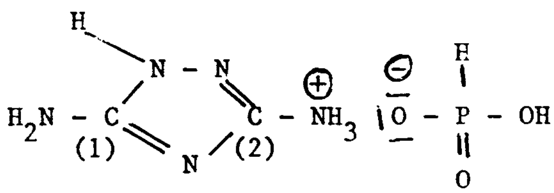

$\delta$ = 153,4 ppm $C_1$, $C_2$

EXEMPLE 6

Phosphite d'amino-3 triazole-1,2,4

Dans un appareillage identique à l'exemple 2 on introduit 250 g d'eau et 464 g d'amino-3 triazole-1,2,4 (5,5 moles). On agite la suspension et on introduit goutte à goutte 624,5 g d'une solution aqueuse à 72,5 % $H_3PO_3$ (5,52 moles de $H_3PO_3$).

L'addition s'effectue à température ambiante, la température s'élève à 30 °C. L'addition terminée (4 h30), on maintient la suspension vers 35 °C sous bonne agitation pendant 1 h 30 puis on filtre, essore, lave le gâteau obtenu et sèche à 100 °C sous pression réduite. On obtient 914 g de phosphite d'amino-3

triazole-1,2,4.
Rendement : 84 %
Fusion : 130,9 ° C

Analyse élémentaire

| $C_2H_7N_4O_3P$ | | | | |
|---|---|---|---|---|
| | C | H | N | P |
| % calculé<br>% trouvé | 14,45<br>13,89 | 4,21<br>4,32 | 33,73<br>32,91 | 18,67<br>17,85 |

Spectre infra-rouge

$\nu$ P - H = 2410 $cm^{-1}$
$\nu$ P - OH = 2680 $cm^{-1}$
$\nu$ $NH_3^{\oplus}$ , $NH_2$ = 3140 $cm^{-1}$, 3280 $cm^{-1}$
$\nu$ P = O = 1105 $cm^{-1}$

Spectre RMN du Proton et du $^{13}C$

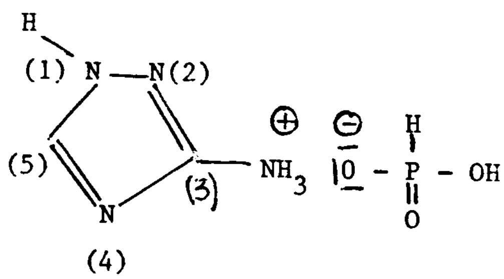

Spectre RMN du $^{31}P$ (Solvant $D_2O$ référence $H_3PO_4$)

$\delta$ = 5,14 ppm

Spectre RMN du proton (Solvant DMSO deutéré)

$\delta$ = 6,88 ppm P - H JP - H = 614 $H_z$

Spectre RMN du $^{13}C$ (Solvant DMSO deutéré)

$\delta$ = 140,73 ppm $C_5$
$\delta$ = 152,76 ppm $C_3$

EXEMPLE 7

Phosphite d'acétoguanamine

Dans un réacteur de 4 l, muni d'une agitation, d'une prise de température, d'un réfrigérant à reflux, on introduit 2 l d'eau et 125 g d'acétoguanamine (1 mole). On agite, puis on introduit goutte à goutte 117,7 g d'une solution aqueuse à 69,7 % de $H_3PO_3$.

L'addition terminée, on porte le milieu réactionnel à 50°C pendant 1 heure, puis on refroidit, filtre et essore. Le produit est lavé, puis séché sous pression réduite vers 100°C.

Analyse élémentaire

| $C_4H_{10}N_5O_3P$ | | | |
|---|---|---|---|
| | C | H | N |
| % calculé<br>% trouvé | 23,18<br>23,28 | 4,83<br>4,75 | 33,81<br>33,64 |

Spectre infra-rouge

ν P - H = 2400 $cm^{-1}$
ν C = N = 1670 $cm^{-1}$

Spectre RMN du carbone $^{13}C$ (Solvant DMSO deuteré)

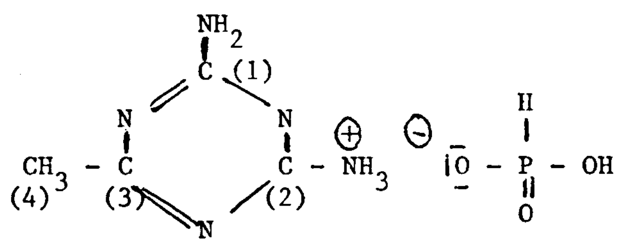

δ = 22,85 ppm $C_4$
δ = 164,67 ppm $C_1$, $C_2$
δ = 171,56 ppm $C_3$

EXEMPLE 8

Phosphite de pipérazine

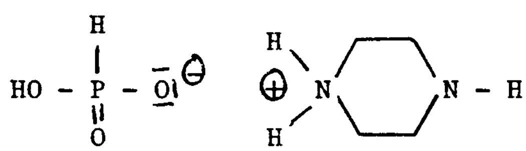

Dans un réacteur de 2 litres équipé comme dans l'exemple 2, on introduit 86 g de pipérazine (1 mole) dans 500 ml d'eau. On agite la solution et on introduit goutte à goutte, en 40 minutes, 116,7 g d'une solution aqueuse de $H_3PO_3$ à 70,25 %.

La température s'élève de 25°C à environ 40°C en fin d'addition. Après refroidissement on concentre partiellement la solution obtenue sous pression réduite.
Le précipité obtenu est filtré, essoré puis séché à 100°C sous pression réduite.
On obtient 127 g de phosphite de pipérazine.
Fusion : 215°C

Spectre infra-rouge

$\nu$ PH = 2320 $cm^{-1}$
$\delta$ $NH_2^{\oplus}$ = 1440 $cm^{-1}$

Spectre RMN du proton(Solvant $D_2O$)

$\delta$ = 6,78 ppm (d) P-H JP-H = 582 $H_z$
$\delta$ = 3,34 ppm (s) -C$\underline{\underline{H}}_2$- (8 protons)

Spectre RMN du $^{13}C$ (Solvant $D_2O$)

$\delta$ = 43,416 ppm

EXEMPLE 9

Diphosphite de pipérazine

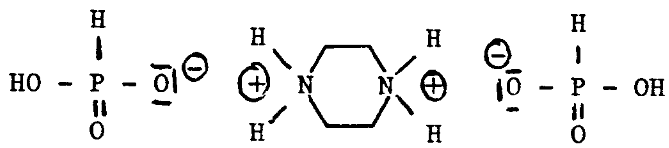

Dans un réacteur de 2 litres équipé comme dans l'exemple 2, on introduit 86 g de pipérazine (1 mole) dans 250 ml d'eau. On agite puis on introduit goutte à goutte en 1 h 30, 233,5 g d'une solution aqueuse de $H_3PO_3$ à 70,25 % (2 moles de $H_3PO_3$ 100 %).

La température s'élève à environ 45/50 ° C.

On maintient l'agitation pendant 1 heure puis, après refroidissement, on concentre à sec sous pression réduite, puis sèche à 100 ° C.

On obtient le diphosphite de pipérazine.

Fusion : 138 ° C

Spectre infra rouge

$\nu$ PH = 2360 $cm^{-1}$
$\delta$ $NH_2^{\oplus}$ = 1450 $cm^{-1}$

Spectre RMN du proton (Solvant $D_2O$)

$\delta$ = 6,85 ppm (d) P-H JP-H = 627 $H_z$
$\delta$ = 3,57 ppm (s) -C$\underline{\underline{H}}_2$- (8 protons)

Spectre RMN du $^{13}C$ (Solvant $D_2O$)

$\delta$ = 42,82 ppm

L'efficacité des phosphites d'amines utilisés seuls ou associés à du pentaerythritol comme agents améliorant le comportement au feu des polyamides et des polyoléfines a été testée selon les exemples suivants :

EXEMPLE 10 (témoin)

Des granulés de polyamide 11 = grade BMNO commercialisé par la demanderesse et présentant les caractéristiques suivantes : $\eta$ = 1,01, densité = 1,03, fusion = 185 ° C, sont extrudés sur un malaxeur BUSS.

Les granulés obtenus sont moulés par injection à une température de 230°C en éprouvettes sur lesquelles on pratique l'essai UL 94 et la mesure d'indice d'oxygène IO.

EXEMPLE 11

On mélange à sec au tonneau

- 9700 g de granulés de polyamide 11 - type BMNO ayant les mêmes caractéristiques que dans l'exemple 10
- 300 g de phosphite de mélamine.

On alimente avec ce mélange un comalaxeur BUSS type PR 46, dont la température moyenne est de 205/210°C. Les joncs qui en sont extrudés sont refroidis et découpés. Les granulés ainsi obtenus après séchage sont moulés par injection à une température voisine de 225°C.

On pratique sur ces éprouvettes l'essai UL 94 et la mesure d'indice d'oxygène.

EXEMPLES 12, 12 ET 14

Analogues à l'exemple 11 mais avec des doses de phosphite de mélamine différentes.

EXEMPLE 15

On mélange à sec au tonneau :

- 9200 g de granulés de polyamide 11 (grade BMNO)
- 750 g de phosphite de mélamine
- 50 g de pentaerythritol (grade tech. Crystals commercialisé par Celanese Chemical Company)

On opère ensuite comme dans l'exemple 11.

EXEMPLES 16, 17, 18, 19 ET 20

Analogues à l'exemple 15 mais avec des doses de phosphite de mélamine et de pentaerythritol différentes.

EXEMPLE 21

Analogue à l'exemple 20 sauf qu'on remplace le phosphite de mélamine par du phosphite de benzoguanamine.

EXEMPLE 22

On mélange à sec au tonneau :

- 8900 g de granulés de polyamide 11 (grade BMNO)
- 800 g de phosphite de guanazole
- 300 g de pentaerythritol

On opère ensuite comme dans l'exemple 11.

EXEMPLE 23 (Témoin)

Préparation d'un polyamide 12 selon un mode opératoire décrit par la demanderesse. Dans un autoclave en inox on introduit :

- 30 kg de lauryllactame
- 3 kg d'eau
- et 255 g d'acide dodécanedioïque.

On monte en température à 280°C sous pression élevée de 25 à 30 bars qu'on maintient 2 heures. On détend en laissant la température à 250°C et on continue la polymérisation à la pression atmosphérique et sous faible balayage d'azote pendant le temps nécessaire pour obtenir un polymère de viscosité inhérente de 1,01 qu'on extrude hors de l'autoclave (par pression d'azote) sous forme de joncs qui sont solidifiés par refroidissement dans l'eau.

Ces joncs sont ensuite découpés en granulés qui sont séchés. Les granulés ainsi obtenus sont moulés par injection comme on le décrit dans l'exemple 10.

EXEMPLE 24

On mélange à sec au tonneau :
- 8200 g de granulés de polyamide 12 obtenus dans l'exemple 23,
- 1500 g de phosphite de mélamine,
- 300 g de pentaerythritol.

On opère ensuite comme dans l'exemple 11.

EXEMPLE 25

Analogue à l'exemple 24 sauf qu'on remplace le phosphite de mélamine par du phosphite d'amino-3 triazole-1,2,4.

EXEMPLE 26

On mélange à sec au tonneau :
- 8950 g d'un polyamide 6 - grade RMN CD, commercialisé par la demanderesse sous le nom D'ORGATER,
- 1000 g de phosphite de mélamine,
- 50 g d'Irganox 1098 (bis[ditertiobutyl-3,5 hydroxy-4, phényl propionyl]-N, N' hexamethylène diamine).

On alimente avec ce mélange un malaxeur BUSS, type PR 40/70 dont les températures moyennes sont comprises entre 230-235 ° C. Les joncs sont refroidis et découpés en granulés.

Les granulés sont séchés puis moulés par injection à une température comprise entre 250/260 ° C en éprouvettes normalisées pour effectuer l'essai UL 94 et mesurer l'indice d'oxygène IO.

Les résultats obtenus sont reportés dans le tableau A. Ces exemples montrent bien que les phosphites d'amines seuls, et mieux associés à du pentaerythritol améliorent le comportement au feu des polyamides.

Les exemples ci-après montrent l'efficacité du système intumescent phosphites d'amines-pentaerythritol pour améliorer le comportement au feu des polyoléfines, en particulier, du polypropylène.

EXEMPLE 27

A partir d'une poudre de polypropylène - PK 1060P, commercialisée par HOESCHT, on moule des éprouvettes normalisées par compression sur presse MINIMATIC pour réaliser l'essai UL 94 et la mesure de l'indice d'oxygène.

EXEMPLE 28

On mélange à sec au tonneau :
- 5450 g de poudre de polypropylène PK 1060P
- 3500 g de phosphite de mélamine
- 1050 g de pentaerythritol.

On alimente un malaxeur BUSS, type PR 46, dont la température moyenne est de 175 ° C. Les joncs qui en sont extrudés sont refroidis et découpés en granulés qui sont moulés par compression sur presse MINIMATIC à une température voisine de 190 ° C.

EXEMPLES 29, 30, 31 ET 32

Analogues à l'exemple 28, mais les doses de phosphite de mélamine et de pentaerythritol sont différentes. L'extrusion a été réalisée sur extrudeuse COLLIN, type ZK 50, à une température moyenne de 170/180 ° C.

EXEMPLE 33

Analogue à l'exemple 30, sauf qu'on remplace le phosphite de melamine par le phosphite d'acétoguanamine.

EXEMPLE 34

Des granulés de polypropylène - type 3050 MN1 - commercialisés par la demanderesse sont extrudés sur malaxeur BUSS, type PR 40/70.

Les granulés obtenus sont moulés par injection en éprouvettes normalisées sur lesquelles on pratique l'essai UL 94 et la mesure de l'indice d'oxygène.

EXEMPLE 35

On mélange à sec au tonneau :
- 6000 g de granulés de prolypropylène 3050 NM1
- 3000 g de phosphite de mélamine.

On alimente avec ce mélange un malaxeur BUSS - type PR 40/70 - en tête de vis malaxeuse, la poudre de pentaerythritol (1000 g) est introduite au moyen d'un doseur SODER en milieu de vis malaxeuse avec un débit tel, qu'on ait dans la composition finale un pourcentage égal à 10 % (en poids). On procède ensuite comme dans l'exemple 27.

EXEMPLE 36

Analogue à l'exemple 35, sauf que les pourcentages de phosphite de mélamine et de pentaerythritol sont différents.

Les résultats obtenus sont reportés dans le tableau B.

Ces exemples montrent bien l'efficacité des phosphites d'amines dans le système intumescent pour améliorer le comportement au feu du polypropylène.

Au cours des essais feu on observe bien la formation d'une "meringue" qui joue le rôle d'isolant ralentissant la libération des gaz inflammables par la masse chauffée.

TABLEAU A

| EXEMPLES / Formulation | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|
| Polyamide 11 | 100 | 97 | 92,5 | 90 | 87,5 | 92 | 91,5 | 91 | 90 | 87,8 | 88 | 88 | 89 | | | | |
| Polyamide 12 | | | | | | | | | | | | | | 100 | 82 | 82 | |
| Polyamide 6 | | | | | | | | | | | | | | | | | 89,5 |
| Phosphite de mélamine | | 3 | 7,5 | 10 | 12,5 | 7,5 | 7,5 | 7,5 | 7,5 | 9,2 | 10 | | | | 15 | | 10 |
| Phosphite de guanazole | | | | | | | | | | | | | 8 | | | | |
| Phosphite d'amino-3triazole | | | | | | | | | | | | | | | | 15 | |
| Phosphite de benzoguanamine | | | | | | | | | | | | 10 | | | | | |
| Pentaerythritol | | | | | | 0,5 | 1 | 1,5 | 2,5 | 3 | 2 | 2 | 3 | | 3 | 3 | |
| IO (%) | 23 | 25 | 26,5 | 28,5 | 29,5 | 28 | 30 | 31,5 | 31 | 30 | 30,1 | 30,4 | 30 | 24 | 27,3 | 30 | 31 |
| ESSAI UL 94 ép. 1,6 mm temps combustion (en sec) | | | 0,7 | 0,4 | 0,6 | 0,5 | | 0 | 0,2 | 0 | 0,9 | 0 | 0,2 | | 0 | 0,3 | 0 |
| Classement | V2 | | V2 | V2 | V2 | V2 | | VO | VO | VO | V2 | VO | V2 | V2 | V2 | VO | V2 |
| ép. 3,2 mm temps combustion (en sec) | | 2,2 | 1 | 1,2 | 0,6 | 1,3 | 0,1 | 0,9 | 1,7 | 1,7 | 0,5 | 0,2 | 0,8 | | 0,8 | 1 | 0 |
| Classement | V2 | V2 | V2 | V2 | V2 | V2 | V2 | VO | V2 | V2 | V2 | VO | V2 | V2 | V2 | V2 | V2 |

TABLEAU B

| Formulation / EXEMPLES | 27 | 28 | 29 | 30 | 31 | 32 | 33 | 34 | 35 | 36 |
|---|---|---|---|---|---|---|---|---|---|---|
| Polypropylène - PK 1060 P | 100 | 54,5 | 60 | 65 | 70 | 75 | 65 | | | |
| Polypropylène - 3050 MN | | | | | | | | 100 | 60 | 70 |
| Phosphite de mélamine | | 35 | 30 | 26,25 | 22,5 | 18,75 | | | 30 | 22,5 |
| Phosphite d'acétoguanamine | | | | | | | 26,25 | | | |
| Pentaerythritol | | 10,5 | 10 | 8,75 | 7,5 | 6,25 | 8,75 | | 10 | 7,5 |
| IO (%) | 17,3 | 40,5 | 38 | 35 | 31,5 | 30 | 32 | 17,5 | 38,5 | 33,7 |
| Classement à l'essai UL 94 : | | | | | | | | | | |
| 0,8 mm d'épaisseur | | VO | | | | | | | VO | |
| 1,6 mm d'épaisseur | | VO | VO | VI | VI | NC | | | VO | |
| 3,2 mm d'épaisseur | NC | VO | VO | VO | VO | VI | VO | NC | VO | VO |

## Revendications

**1.** Sels d'acide phosphoreux de formule (I)

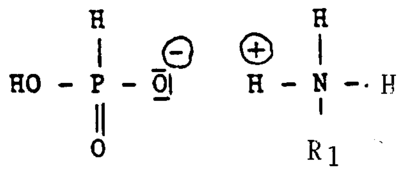

(I)

caractérisés en ce que $R_1$ est un hétérocycle contenant des atomes d'azote et pouvant être substitué par des groupements amino, des halogènes, des radicaux aliphatiques ayant jusqu'à 10 atomes de carbone, des radicaux phényles. $R_1$ étant choisi parmi les radicaux de structure : - s-triazinique,

- 1,2,4 triazolyle,
- benzimidazolyle,
- heptazinique,
- 1,3 diazinique (pyrimidique)

**2.** Produits selon la revendication 1, caractérisé en ce qu'ils sont de formules suivantes :

- le phosphite de mélamine :

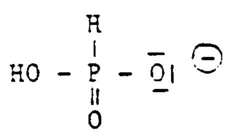

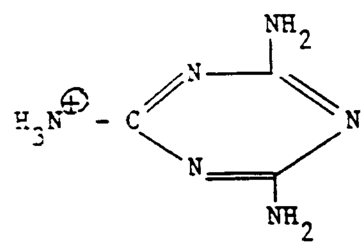

- le phosphite de melem :

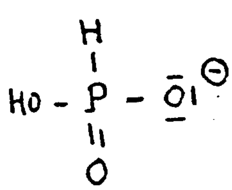

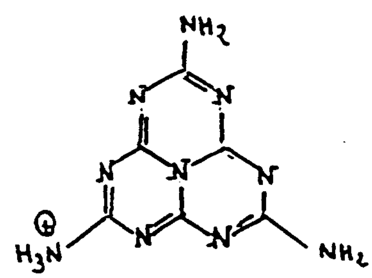

- le phosphite de benzoguanamine :

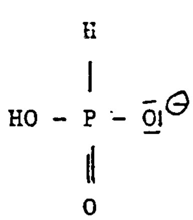

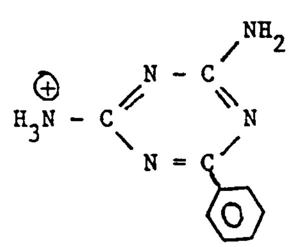

- le phosphite d'acétoguanamine :

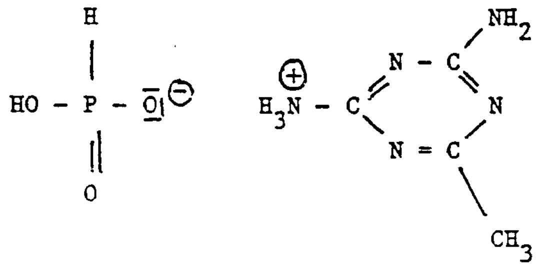

- le phosphite d'amino-3 triazole-1,2,4 :

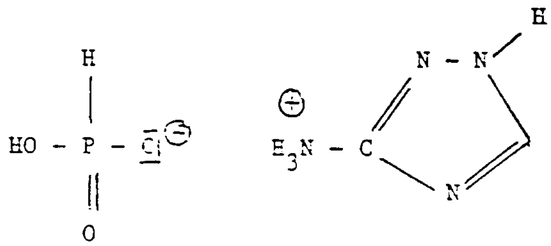

- le phosphite d'amimo-4 triazole-1,2,4 :

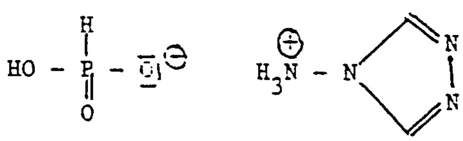

- le phosphite de guanazole :

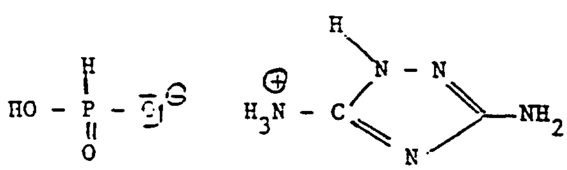

- le phosphite de benzimidazole :

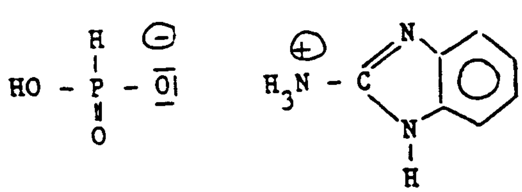

**3.** Produits de formule

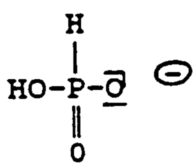 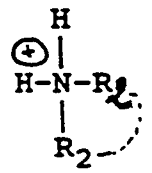 (I-a)

caractérisés en ce que les deux substituants $R_2$ sont des restes aliphatiques identiques ayant jusqu'à 4 atomes de carbone pouvant être substitués par des halogènes ou des groupements amino, et que ces restes $R_2$ sont pontés par un reste > NH ou un reste méthylénique - $CH_2$-.

**4.** Produits selon la revendication 3, caractérisés en ce que les restes $R_2$ sont tels qu'on ait une structure piperazinique, ou pipéridinique et sont de préférence :

- le phosphite de piperazine

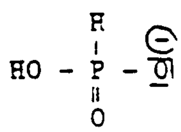 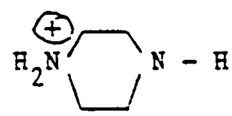

- le phosphite de pipéridine

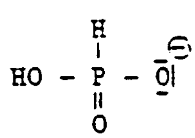 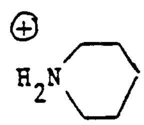

**5.** Sels d'acide phosphoreux de formule (II)

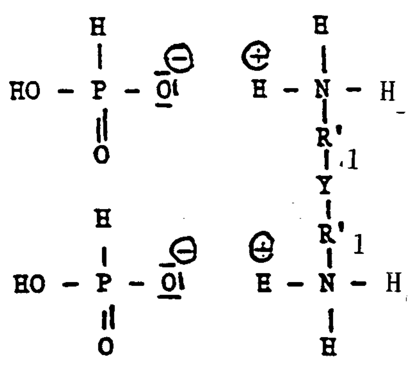 (II)

caractérisés en ce que R'$_1$ un radical de structure triazinique pouvant être substitué par des groupements amino, des halogènes ou des radicaux aliphatiques ayant jusqu'à 10 atomes de carbone,

Y est un groupement :

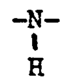

ou -NH-$(CH_2)n$ - NH-avec n compris entre 2 et 6, ou

-NH $(CH_2)_2$ - NH - $(CH_2)_2$ - NH-

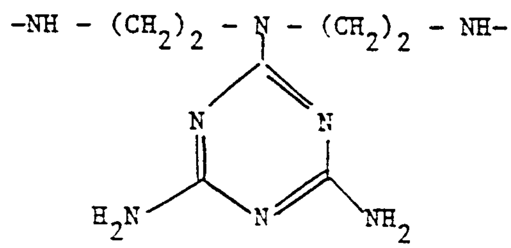

ou une simple liaison
et que le produit est de préférence :

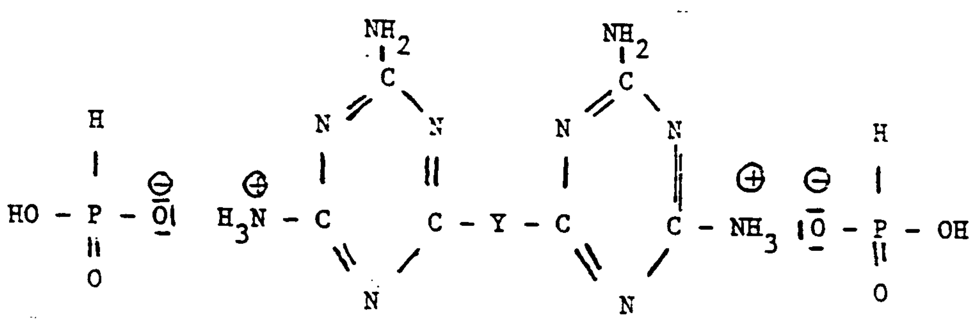

**6.** Produits selon la revendication 5, caractérisés en ce qu'ils sont de préférence :

- le diphosphite de mélam :

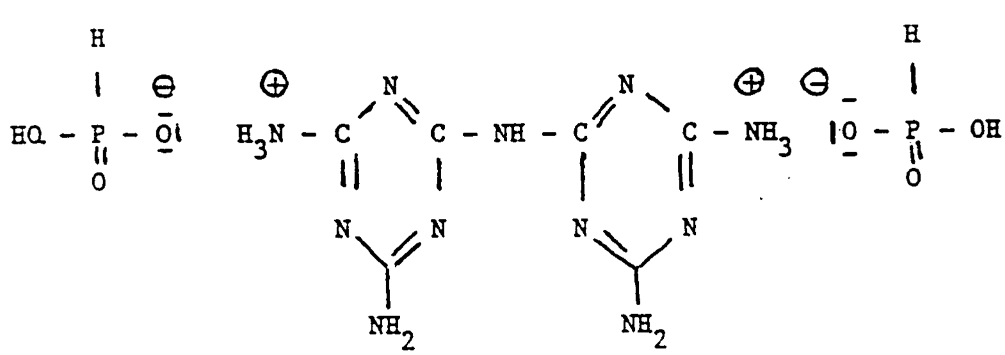

- le diphosphite d'éthylène dimélamine :

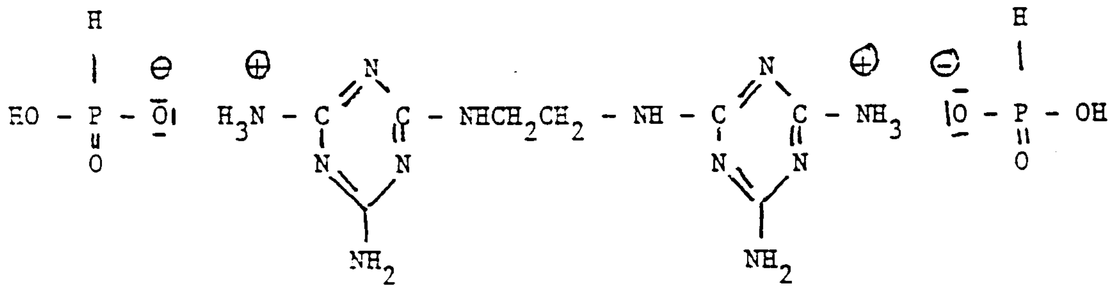

- le diphosphite de diethylènetrimelamine :

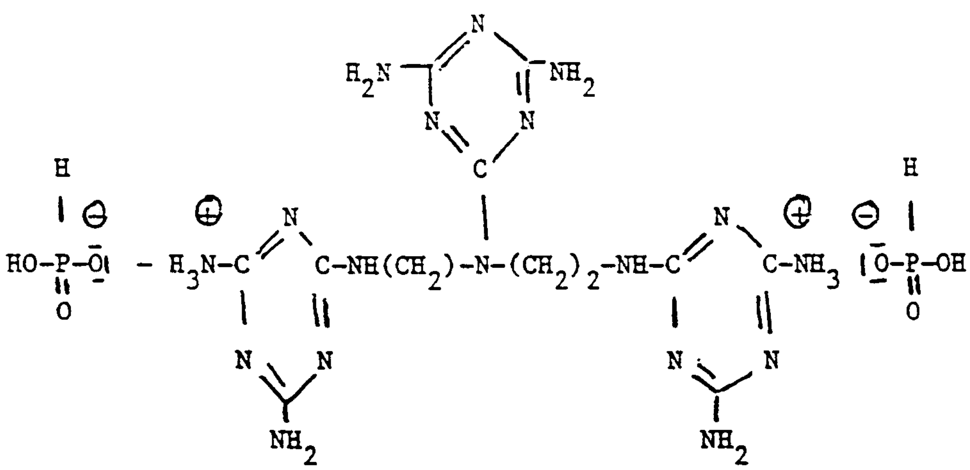

**7.** Le diphosphite de pipérazine :

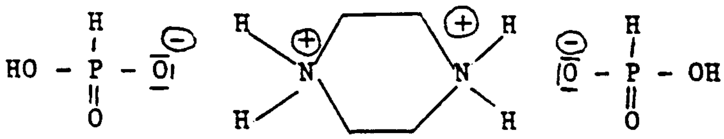

**8.** Application des produits selon l'une des revendications 1 à 7 à l'ignifugation des matières plastiques.

**9.** Procédé selon la revendication 8 caractérisé en ce qu'on utilise de préférence le phosphite de mélamine.

**10.** Procédé selon la revendication 8 ou 9, caractérisé en ce qu'on utilise avantageusement 1 à 20 % en poids de phosphites par rapport aux polyamides ignifugées et de préférence 3 à 12 %.

**11.** Procédé selon la revendication 8 ou 9, caractérisé en ce qu'on utilise avantageusement 10 à 60 % en poids de phosphites par rapport aux polyoléfines ignifugées et de préférence 25 à 35 %.

**12.** Procédé selon l'une des revendications 8 à 11, caractérisé en ce qu'on utilise les phosphites en association avec un ou plusieurs composés polyhydroxylés choisis parmi l'érythrytol, le sorbitol, le mannitol, le dianhydrosorbitol, l'anhydroerythritol, la mono- di ou tripentaerythrite.

**13.** Procédé selon la revendication 12 caractérisé eu ce que le composé polyhydroxylé est de préférence du pentaerythritol.

**14.** Procédé selon l'une des revendications 12 ou 13, caractérisé eu ce qu'on utilise avantageusement 0,2 à 10 % en poids de composés polyhydroxylés par rapport au polyamide ignifugé et de préférence 1 à 3 %.

**15.** Procédé selon l'une des revendications 12 ou 13 caractérisé en ce qu'on utilise avantageusement dans les polyoléfines le composé polyhydroxylé en proportion telle que le rapport molaire

$$\frac{\text{phosphite d'amine}}{\text{composé hydroxylé}}$$

est compris entre 1 et 7 et de préférence entre 2 et 4.

**16.** Procédé selon la revendication 15 caractérisé en ce que la quantité de phosphite d'amine plus composé polyhydroxylé est avantageusement compris entre 20 et 60 % en poids par rapport aux résines ignifugées et de préférence entre 25 et 35 %.

**17.** Matières plastiques ignifugées selon le procédé des revendications 8 à 16.

**18.** Application des phosphites de morpholine, de cyclohexylamine et d'aniline à l'ignifugation des matières plastiques.

**Claims**

**1.** Phosphorous acid salts of formula (I)

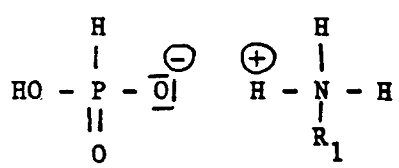

(I)

characterised in that $R_1$ is a heterocyclic ring containing nitrogen atoms and capable of being substituted by amino groups, halogens, aliphatic radicals containing up to 10 carbon atoms, or phenyl radicals, $R_1$ being chosen from radicals with the following structure:

- s-triazine,
- 1,2,4-triazolyl,
- benzimidazolyl,
- heptazine, and
- 1,3-diazine (pyrimidine).

**2.** Products according to Claim 1, characterised in that they are of the following formulae:

- melamine phosphite:

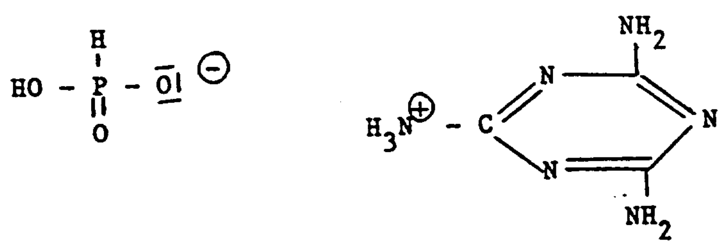

- melem phosphite:

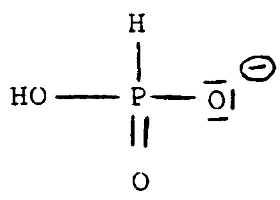 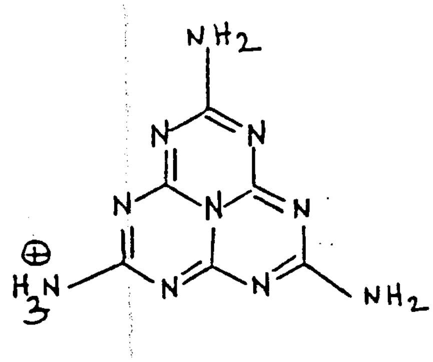

- benzoguanamine phosphite:

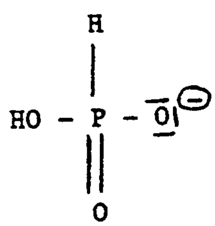 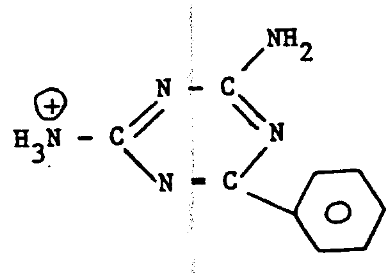

- acetoguanamine phosphite:

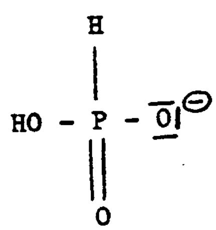 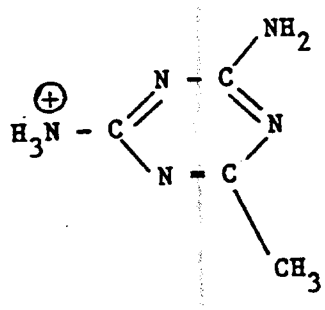

- 3-amino-1,2,4-triazole phosphite:

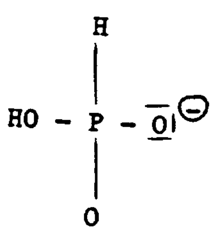 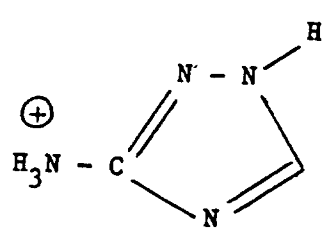

- 4-amino-1,2,4-triazole phosphite:

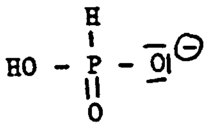

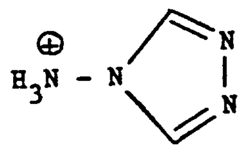

- guanazole phosphite:

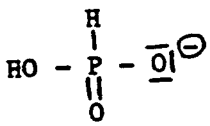

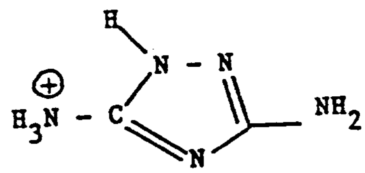

- benzimidazole phosphite:

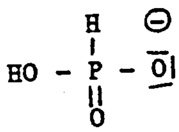

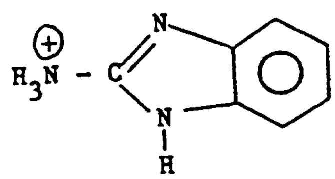

**3.** Products of formula

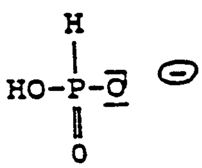

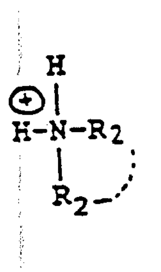

(Ia)

characterised in that the two substituents $R_2$ are identical aliphatic residues containing up to 4 carbon atoms capable of being substituted by halogens or amino groups, and in that these residues $R_2$ are bridged by a >NH residue or a methylene residue $-CH_2-$.

**4.** Products according to Claim 3, characterised in that the residues $R_2$ are such that a piperazine or piperidine structure is present, and are preferably;

- piperazine phosphite

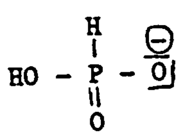

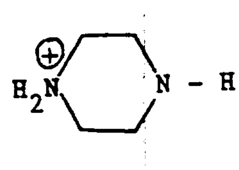

- piperidine phosphite

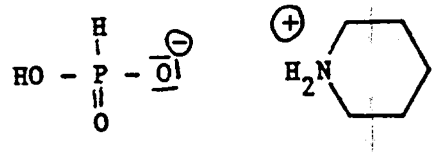

**5.** Phosphorous acid salts of formula (II)

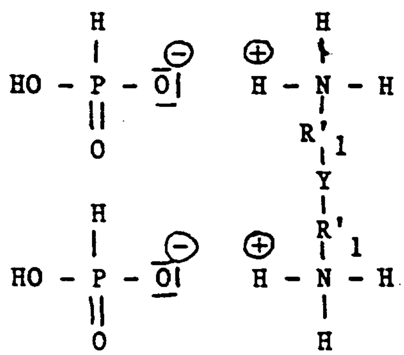

(II)

characterised in that $R_1$ is a radical of triazine structure capable of being substituted by amino groups, halogens or aliphatic radicals containing up to 10 carbon atoms

Y is a group:

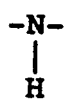

or $-NH-(CH_2)_n-NH-$ with n being between 2 and 6, or

$-NH\ (CH_2)_2 - NH - (CH_2)_2 - NH-$

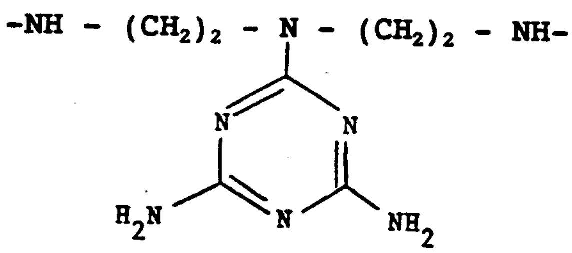

or a single bond

and in that the product is preferably:

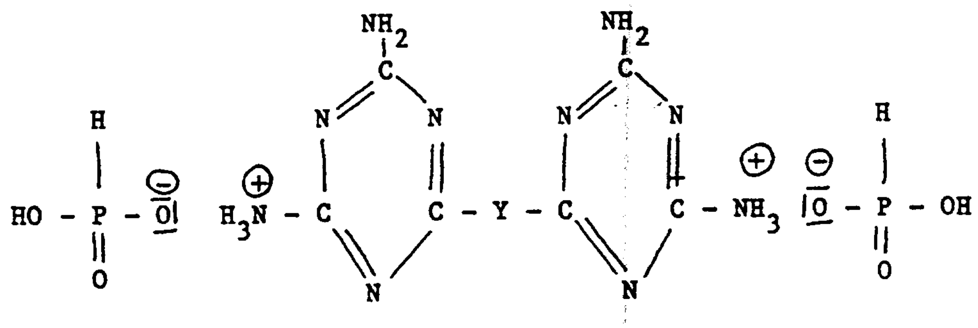

**6.** Products according to Claim 5, characterised in that they are preferably:

- melam diphosphite:

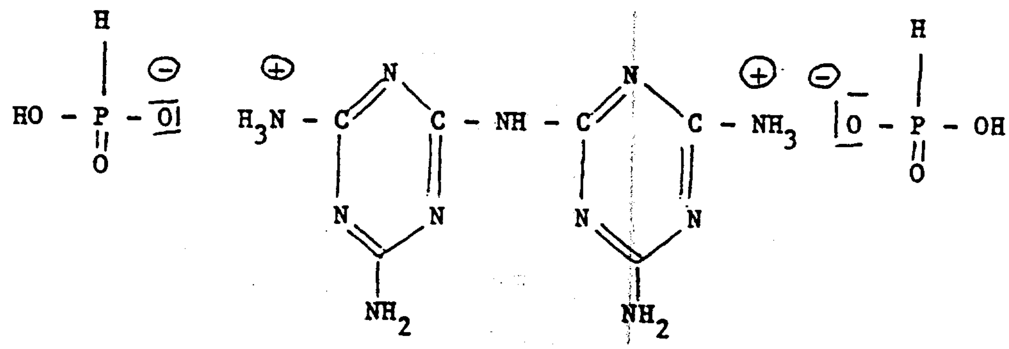

- ethylenedimelamine diphosphite:

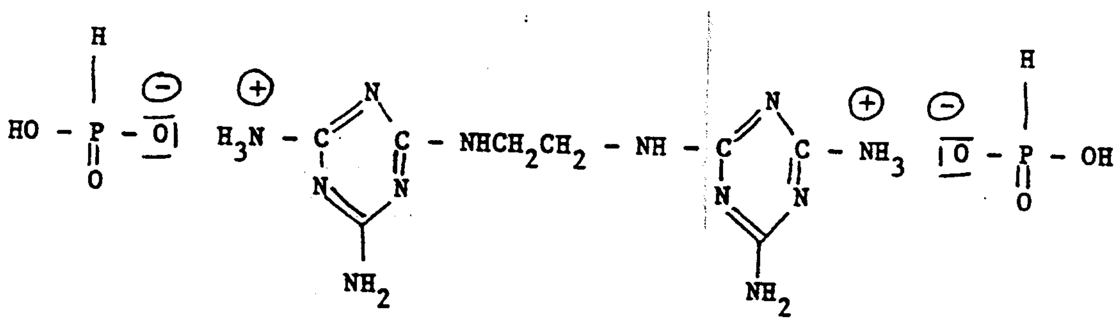

- diethylenetrimelamine diphosphite:

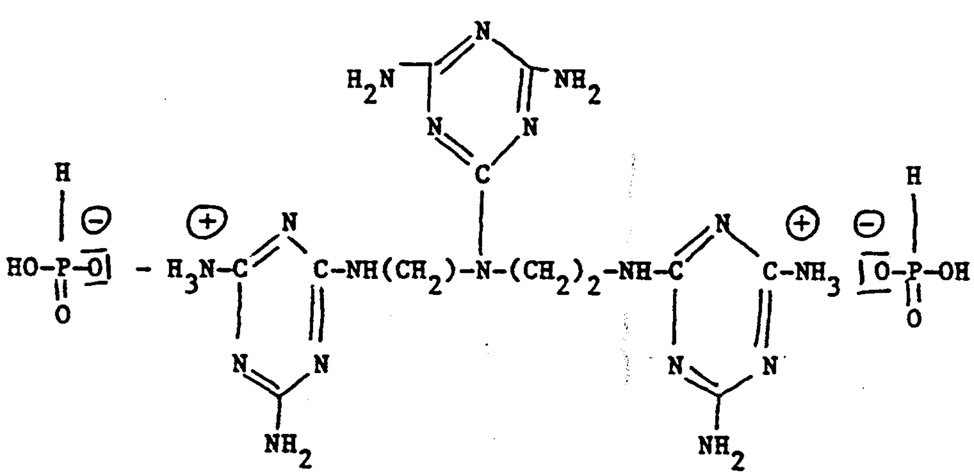

**7.** Piperazine diphosphite:

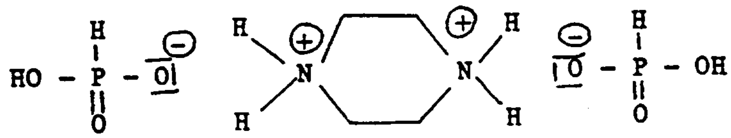

**8.** Application of the products according to one of Claims 1 to 7 to the fireproofing of plastics.

**9.** Process according to Claim 8, characterised in that melamine phosphite is preferably employed.

**10.** Process according to Claim 8 or 9, characterised in that 1 to 20 % by weight of phosphites relative to the fireproofed polyamides, and preferably 3 to 12 %, are advantageously employed.

**11.** Process according to Claim 8 or 9, characterised in that 10 to 60 % by weight of phosphites relative to the fireproofed polyolefins, and preferably 25 to 35 %, are advantageously employed.

**12.** Process according to one of Claims 8 to 11, characterised in that the phosphites are employed in combination with one or more polyhydroxylated compounds chosen from erythritol, sorbitol, mannitol, dianhydrosorbitol, anhydroerythritol and mono-, di- or trierythritol.

**13.** Process according to Claim 12, characterised in that the polyhydroxylated compound is preferably pentaerythritol.

**14.** Process according to either of Claims 12 and 13, characterised in that 0.2 to 10 % by weight of polyhydroxylated compounds relative to the fireproofed polyamide, and preferably 1 to 3 %, are advantageously employed.

**15.** Process according to either of Claims 12 and 13, characterised in that the polyhydroxylated compound is advantageously employed in the polyolefins in such proportion that the molar ratio amine phosphite/hydroxylated compound is between 1 and 7 and preferably between 2 and 4.

**16.** Process according to Claim 15, characterised in that the quantity of amine phosphite plus polyhydroxylated compound is advantageously between 20 and 60 % by weight relative to the fireproofed resins, and preferably between 25 and 35 %.

**17.** Plastics fireproofed according to the process of Claims 8 to 16.

**18.** Application of morpholine, cyclohexylamine and aniline phosphites, to the fireproofing of plastics.

**Patentansprüche**

**1.** Salze der phosphorigen Säure der Formel (1)

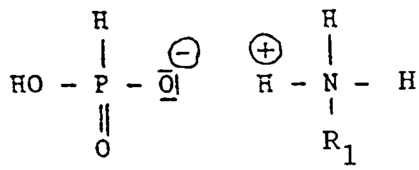

dadurch gekennzeichnet, daß $R_1$ ein Heterocyclus ist, der Stickstoffatome enthält und durch Aminogruppen, Halogen, aliphatische Reste mit bis zu 10 Kohlenstoffatomen und Phenylreste substituiert sein kann und wobei $R_1$ unter den Resten der folgenden Struktur ausgewählt ist,

- s-Triazinyl,

- 1,2,4 Triazolyl,
- Benzimidazolyl,
- Heptazin
- 1,3 Diazinyl (Pyrimidinyl)

**2.** Produkte nach Anspruch 1, dadurch gekennzeichnet, daß sie die folgenden Formel haben

- Melaminphosphit

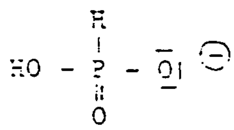

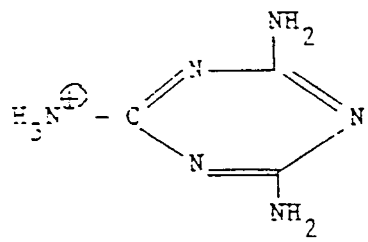

- Melemphosphit

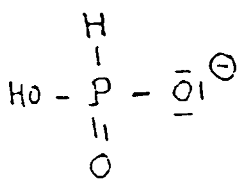

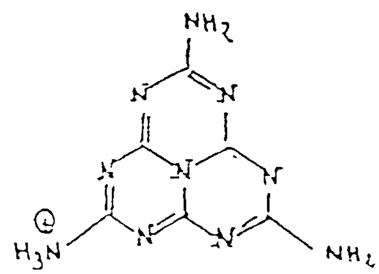

- Benzoguanaminphosphit

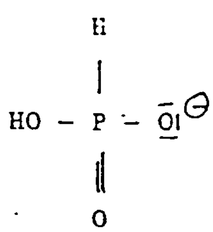

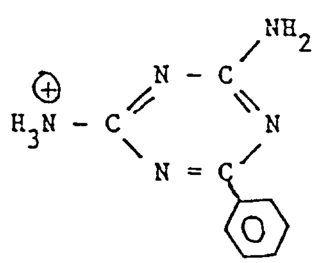

- Acetoguanaminphosphit

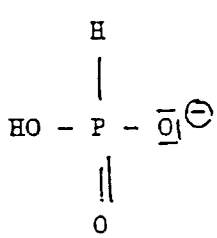

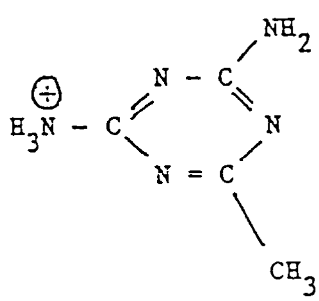

- 3-Amino-1,2,4-triazolphosphit

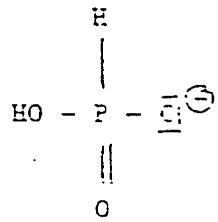

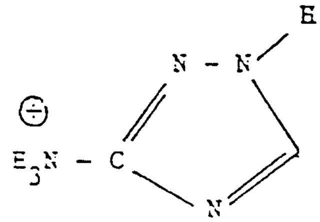

- 4-Amino-1,2,4-triazolphosphit

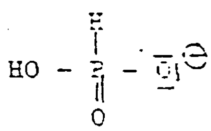

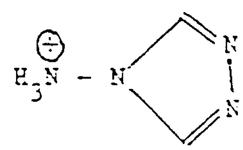

- Guanazolphosphit

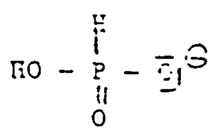

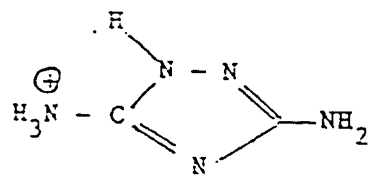

- Benzimidazolphosphit

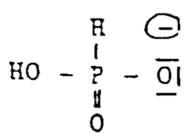

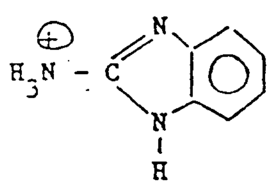

**3.** Produkte der Formel

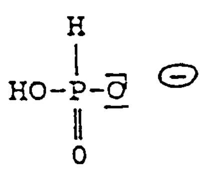

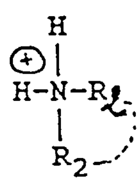

(I-a)

dadurch gekennzeichnet, daß die beiden Substituenten $R_2$ identische aliphatische Reste mit bis zu 4 Kohlenstoffatomen bedeuten, die durch Halogene oder Aminogruppen substituiert sein können und daß diese Reste $R_2$ durch einen Rest > NH oder einen Methylenrest $CH_2$ verbunden sind.

**4.** Produkte nach Anspruch 3, dadurch gekennzeichnet, daß die Reste $R_2$ derart sind, daß man eine Piperazin- oder Piperidinstruktur und vorzugsweise

- Piperazinphosphit

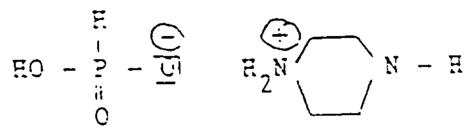

- Piperidinphosphit

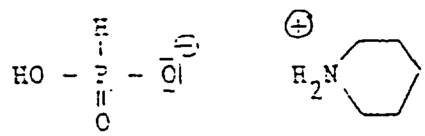

erhält.

**5.** Salze der phosphorigen Säure der Formel (II)

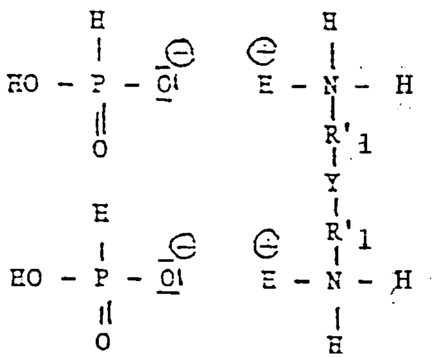

(II)

dadurch gekennzeichnet, daß der Rest $R_1$ eine Triazinstruktur hat und durch Aminogruppen, Halogen oder aliphatische Reste mit bis zu 10 Kohlenstoffatomen substituiert sein kann und
Y die Gruppierung

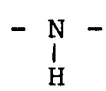

oder $-NH-(CH_2)_n-NH-$ mit n gleich 2 bis 6, oder

$- NH (CH_2)_2 - NH - (CH_2)_2 - NH -$

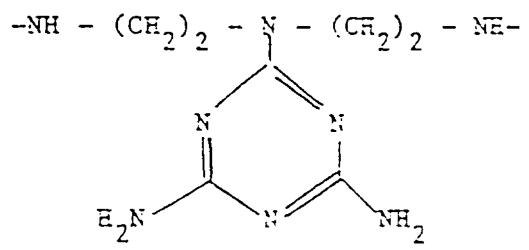

oder eine einfache Bindung ist und daß das Produkt vorzugsweise

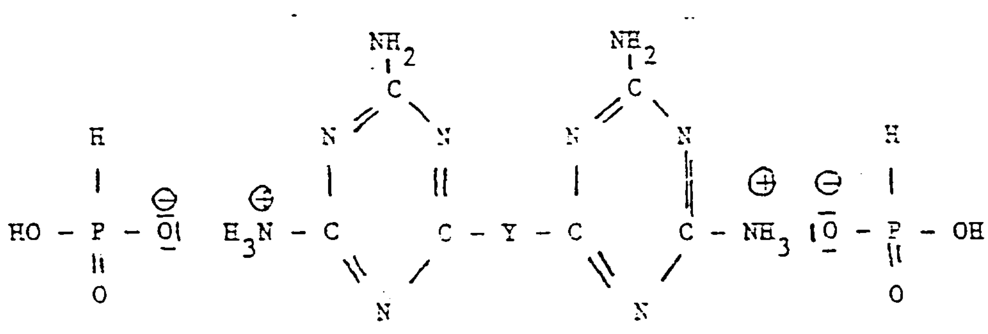

ist.

6. Produkte nach Anspruch 5, dadurch gekennzeichnet, daß es sich vorzugsweise um die folgenden handelt :
   - Melamdiphosphit

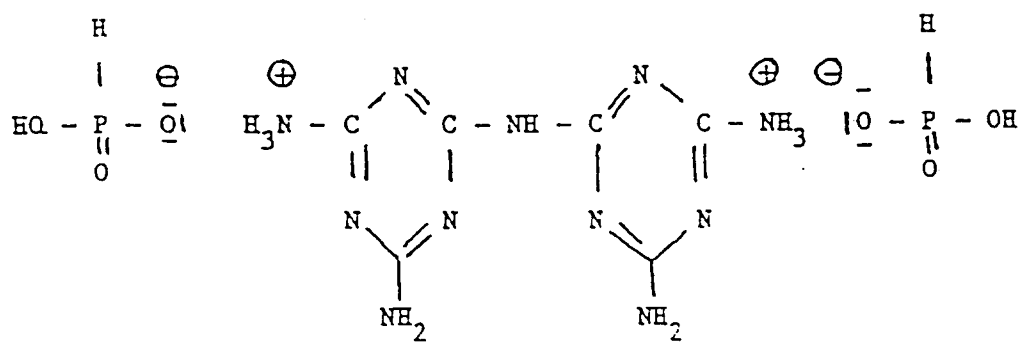

- Ethylendimelamindiphosphit

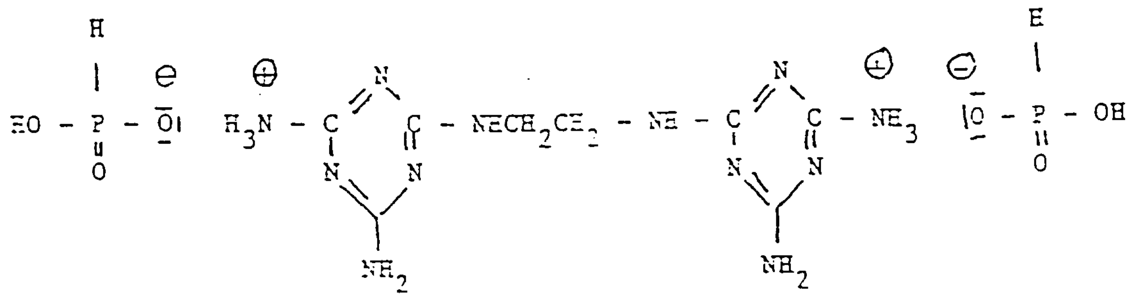

- Diethylentrimelamindiphosphit

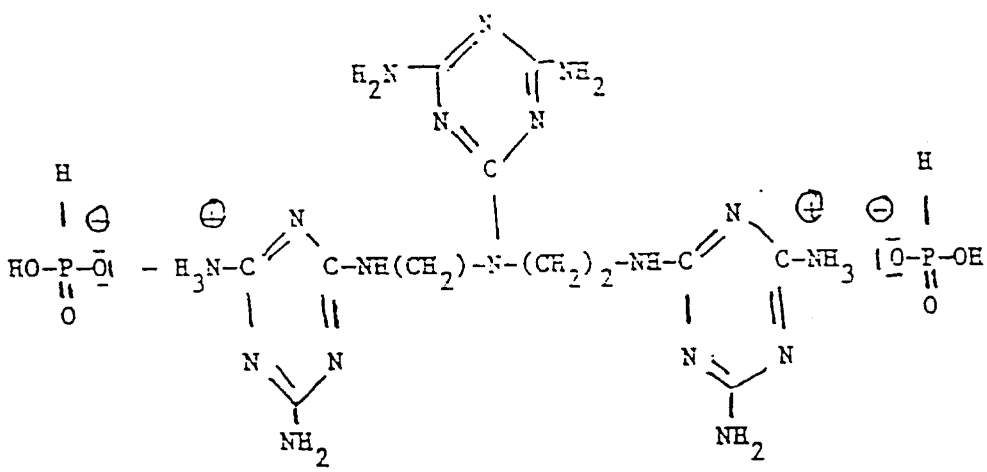

**7.** Piperazindiphosphit :

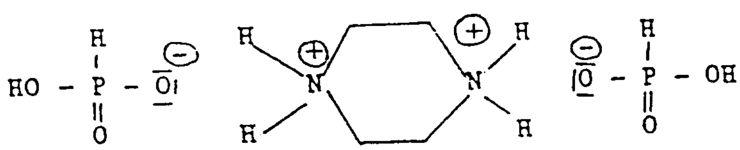

**8.** Verwendung von Produkten nach einem der Ansprüche 1 bis 7 zum Brandschutz von Kunststoffen.

**9.** Verfahren nach Anspruch 8, dadurch gekennzeichnet, daß man vorzugsweise Melaminphosphit verwendet.

**10.** Verfahren nach Anspruch 8 oder 9, dadurch gekennzeichnet, daß man vorzugsweise 1 bis 20 Gew.% und insbesondere 3 bis 12 Gew.% der Phosphite, bezogen auf die flammwidrig gemachten Polyamide verwendet.

**11.** Verfahren nach Anspruch 8 oder 9, dadurch gekennzeichnet, daß man 10 bis 60, vorzugsweise 25 bis 35 Gew.% der Phosphite, bezogen auf die flammwidrig gemachten Polyolefine verwendet.

**12.** Verfahren nach einem der Ansprüche 8 bis 11, dadurch gekennzeichnet, daß man die Phosphite in Verbindung mit einer oder mehreren Polyhydroxyverbindungen verwendet, die ausgewählt sind unter

Erythrose, Sorbit, Mannit, Dianhydrosorbit, Anhydroerythrose, Mono,-Di oder Tripentaerythrit.

**13.** Verfahren nach Anspruch 12, dadurch gekennzeichnet, daß die polyhydroxylierte Verbindung vorzugsweise Pentaerythrit ist.

**14.** Verfahren nach einem der Ansprüche 12 oder 13, dadurch gekennzeichnet, daß man 0,2 bis 10 vorzugsweise 1 bis 3 Gew.% der polyhydroxylierten Verbindungen, bezogen auf das flammwidrig gemachte Polyamid verwendet.

**15.** Verfahren nach einem der Ansprüche 12 oder 13, dadurch gekennzeichnet, daß man die Polyhydroxylverbindung in den Polyolefinen in einer solchen Menge verwendet, daß das Molverhältnis Aminphosphit/Hydroxylverbindung zwischen 1 und 7 und vorzugsweise zwischen 2 und 4 liegt.

**16.** Verfahren nach Anspruch 15, dadurch gekennzeichnet, daß die Menge des Aminphosphits plus Polyhydroxylverbindung zwischen 20 und 60 vorzugsweise zwischen 25 bis 35 Gew.%, bezogen auf die flammwidrig gemachten Harze, beträgt.

**17.** Kunststoffe, die nach dem Verfahren der Ansprüche 8 bis 16 flammwidrig gemacht wurden.

**18.** Verwendung der Phosphite von Morpholin, Cyclohexylamin und Anilin zum Brandschutz von Kunststoffen.